# DEMANDE DE BREVET EUROPEEN

(11) **EP 4 722 390 A1**
(43) Date de publication de la demande: **08.04.2026**
(21) Numéro de dépôt: 24306626.3
(22) Date de dépôt: 04.10.2024
(51) Int. Cl.: C12Q 1/689, C12N 1/20

(54) **BACTERIE DU GENRE BREVUNDIMONAS ET SES UTILISATIONS COSMETIQUES**

(71) Demandeur: Laboratoires M & L, 04100 Manosque En Provence (FR); Université d'Aix Marseille, 13007 Marseille (FR); Assistance Publique - Hôpitaux de Marseille, 13005 Marseille (FR)
(72) Inventeur: Cenizo, Valérie, 13650 MEYRARGUES (FR); Maux, Mélinda, 04100 MANOSQUE (FR); Portes, Pascal, 13540 PUYRICARD (FR); Lemaire, Géraldine, 04220 CORBIERE EN PROVENCE (FR); Rivoire, Stéphanie, 04100 MANOSQUE (FR); Boxberger, Manon, 13010 MARSEILLE (FR); La Scola, Bernard, 13109 SIMIANE-COLLONGUE (FR); Cassir, Nadim, 13016 MARSEILLE (FR)
(74) Mandataire: Cabinet Becker et Associés

(57) **Abrégé**

La présente invention concerne une nouvelle bactérie du genre *Brevundimonas.* Une méthode de criblage de substances candidates pour leur effet cosmétique est également proposée.

## Description

La présente invention concerne l'utilisation cosmétique d'une espèce bactérienne du genre *Brevundimonas* dont une souche a nouvellement été isolée.

### Etat de la technique

La peau est un écosystème habité par une pléthore de microorganismes aussi différents que des bactéries, des archées, des champignons ainsi que par des petits arthropodes. Ensemble, ils constituent le microbiote cutané. Ce microbiote est soumis à de nombreux paramètres de variation, à la fois intrinsèques et extrinsèques. Des études récentes ont identifié des corrélations entre les changements de la composition des populations commensales de la peau et l'état physiologique de cet environnement influencé par des facteurs tels que le vieillissement.

Des études de métagénomique ont mis à jour un grand nombre de bactéries, et ont notamment démontré la diminution, au cours du vieillissement, de *Cutibacterium acnés,* une des espèces les plus abondantes du microbiote cutané. Toutefois, ces études de métagénomique sont limitées à la caractérisation des bactéries abondantes, ce qui introduit des biais dans l'analyse de la composition du microbiote. Utilisée seule, la métagénomique ne peut donc suffire à refléter la complexité et la diversité du microbiote cutané.

La culturomique peut aider à pallier les inconvénients de la métagénomique. Cette technique repose sur la multiplication des conditions de culture de micro-organismes, permettant ainsi la croissance d'une plus grande diversité de bactéries, levures, ou champignons dont certains sont considérés comme non cultivables. Ces espèces ne sont pas nécessairement minoritaires dans cet habitat et leur influence est largement méconnue. L'une des principales difficultés de la culturomique cependant est la détermination des conditions d'isolement et de culture favorables à la croissance de certaines souches bactériennes jusqu'à présent méconnues. En effet, selon la zone cutanée considérée, il existe des différences de température, d'humidité ou de pH, qui peuvent influencer la composition bactérienne du microbiote.

### Résumé de l'invention

Les inventeurs sont parvenus à isoler une souche d'une nouvelle espèce bactérienne, dont la présence est corrélée à l'apparition de signes liés à un vieillissement cutané prématuré ou accéléré, en particulier photo-induit.

Les inventeurs ont également remarqué que la présence de cette souche était corrélée à une homogénéité du teint améliorée et une production de sébum accrue, participant à la barrière lipidique, la peau étant plus hydratée et mieux protégée contre les agressions extérieures.

Cette souche a été déposée à la Collection Nationale de Cultures de Microorganismes (CNCM, Institut Pasteur, 25-28 rue du Docteur Roux, 75015 Paris, France) le 13 janvier 2021 sous le numéro **I-5641.**

Un objet de l'invention est une bactérie, ladite bactérie appartenant à l'espèce *Brevundimonas massiliensis sp. nov.* caractérisée en ce que son génome i) présente une identité nucléotidique moyenne (ANI) d'au moins 95% par rapport au génome complet de la souche déposée auprès de la CNCM le 13 janvier 2021 sous le numéro I**-5641** et/ou ii) présente au moins 98,65% d'identité avec la séquence nucléotidique SEQ ID NO : 1 (gène 16S *B.massiliensis sp. nov.*).

De préférence, la bactérie est la souche isolée et déposée à la Collection Nationale de Cultures de Microorganismes (CNCM, Institut Pasteur, 25-28 rue du Docteur Roux, 75015 Paris, France) le 13 janvier 2021 sous le numéro **I-5641.**

L'invention fournit également une méthode de criblage de substances susceptibles de présenter un effet cosmétique, ladite méthode comprenant la mise en contact d'une peau humaine saine avec une substance candidate, et le suivi de la présence et/ou de la quantification d'une bactérie de l'espèce Brevundimonas massiliensis sp. nov. à la surface de la peau, par ce en quoi une substance qui stimule la prolifération de la bactérie à la surface de la peau est identifiée comme susceptible de présenter un effet cosmétique, l'espèce Brevundimonas massiliensis sp. nov. étant caractérisée en ce que son génome i) présente une identité nucléotidique moyenne (ANI) d'au moins 95% par rapport au génome complet de la souche déposée auprès de la CNCM le 13 janvier 2021 sous le numéro I-5641 et/ou ii) présente au moins 98,65% d'identité avec la séquence nucléotidique SEQ ID NO : 1 (gène 16S *B.massiliensis sp. nov.*) ; ladite bactérie étant de préférence la souche déposée à la CNCM le 13 janvier 2021 sous le numéro I-5641.

Plus particulièrement, l'effet cosmétique est un effet de prévention d'un vieillissement cutané prématuré ou accéléré, d'un vieillissement cutané induit par l'environnement, notamment le stress, l'alimentation ou la pollution, d'un vieillissement cutané photo-induit, de prévention de l'apparition de tâches pigmentaires, d'amélioration de l'homogénéité du teint et/ou d'amélioration de la production de sébum.

Un autre objet de l'invention est l'utilisation d'une bactérie de l'espèce Brevundimonas massiliensis sp. nov., comme marqueur d'un vieillissement cutané prématuré ou accéléré, étant entendu que la présence de ladite bactérie est corrélée à une peau protégée contre un vieillissement cutané prématuré ou accéléré ; l'espèce Brevundimonas massiliensis sp. nov. étant caractérisée en ce que son génome i) présente une identité nucléotidique moyenne (ANI) d'au moins 95% par rapport au génome complet de la souche déposée auprès de la CNCM le 13 janvier 2021 sous le numéro I-5641 et/ou ii) présente au moins 98,65% d'identité avec la séquence nucléotidique SEQ ID NO : 1 (gène 16S *B.massiliensis sp. nov.*); ladite bactérie étant de préférence la souche déposée à la CNCM le 13 janvier 2021 sous le numéro I-5641.

### Description détaillée de l'invention

Les inventeurs sont parvenus à isoler une souche appartenant à une nouvelle espèce bactérienne. Par une étude clinique sur volontaires jeunes et âgés, les inventeurs ont mis en évidence la diminution significative de la présence de cette espèce dans des situations de vieillissement cutané prématuré ou accéléré. Sa présence est également associée de manière significative avec un teint homogène et/ou une production de sébum suffisante pour permettre de lutter contre les agressions extérieures.

### Nouvelle espèce bactérienne

Cette bactérie, du genre *Brevundimonas,* est l'espèce *Brevundimonas massiliensis* sp. nov. dont une souche nouvellement isolée a été déposée à la CNCM le 13 janvier 2021 sous le numéro I-5641.

Le génome de la bactérie isolée et déposée à la CNCM le 13 janvier 2021 sous le numéro I-5641 a été entièrement séquencé.

L'appartenance d'une bactérie à cette espèce est définie en ce que son génome i) présente une identité nucléotidique moyenne (ANI) d'au moins 95% par rapport au génome complet de la souche déposée auprès de la CNCM le 13 janvier 2021 sous le numéro I-5641 et/ou ii) présente au moins 98,65% d'identité avec la séquence nucléotidique SEQ ID NO : 1 (gène 16S *B.massiliensis sp. nov.*).

Le terme "identité de séquence" ou "identité" désigne ici le nombre (%) de correspondances (nucléotides identiques) dans les positions d'un alignement de deux séquences nucléotidiques. L'identité de séquence est déterminée en comparant les séquences lorsqu'elles sont alignées de manière à maximiser le chevauchement et l'identité tout en minimisant les écarts de séquence. En particulier, l'identité de séquence peut être déterminée à l'aide d'un certain nombre d'algorithmes mathématiques d'alignement global ou local, en fonction de la longueur des deux séquences. Les séquences de longueur similaire sont de préférence alignées à l'aide d'un algorithme d'alignement global (par exemple, l'algorithme de Needleman et Wunsch ; Needleman et Wunsch, J Mol Biol, 1970 ;48(3):443-53) qui aligne les séquences de manière optimale sur toute la longueur, tandis que les séquences de longueur sensiblement différente sont de préférence alignées à l'aide d'un algorithme d'alignement local (par exemple, l'algorithme de Smith et Waterman (Smith et Waterman, J Mol Biol, 1981 ; 147(1):195-7.) ou l'algorithme d'Altschul (Altschul et al, Nucleic Acids Res. 1997;25(17):3389-402; Altschul et al, FEBS J, 2005;272(20):5101-9). L'alignement aux fins de la détermination du pourcentage d'identité des séquences nucléotidiques peut être réalisé de diverses manières qui relèvent de l'art, par exemple en utilisant des logiciels accessibles au public disponibles sur des sites internet tels que https://blast.ncbi.nlm.nih.gov/Blast.cgi ou http://www.ebi.ac.uk/Tools/emboss/. L'homme du métier peut déterminer les paramètres appropriés pour mesurer l'alignement, y compris les algorithmes nécessaires pour obtenir un alignement maximal sur toute la longueur des séquences comparées.

Le séquençage du gène de l'ARNr 16S est une méthode couramment utilisée pour l'identification de bactéries, plus précisément dans le cadre de la discrimination des espèces bactériennes (Woo, P.C.Y. et al. Clinical Microbiology and Infection, 2008 Volume 14, Issue 10, 908 - 934).

La séquence du gène de l'ARNr 16S peut être amplifiée notamment grâce à des amorces universelles connues de l'homme du métier. De manière préférée, la bactérie contient un gène de l'ARNr 16S présentant au moins 98,65%, de préférence au moins 99%, ou au moins 99,5%, au moins 99,7%, voire au moins 99,8% ou 99,9% d'identité avec la séquence nucléotidique SEQ ID NO : 1.

L'ANI est une méthode de référence de comparaison par paire de séquences bactériennes sur génome entier, fonctionnant à la fois sur génome complet ou partiellement complété, afin de déterminer le niveau de parenté génétique entre les souches bactériennes et définir les espèces bactériennes. En particulier, l'ANI entre deux génomes bactériens est calculée par comparaison par paires de l'ensemble des séquences similaires entre les deux génomes et peut être déterminée, par exemple, en utilisant l'un quelconque des logiciels disponibles publiquement de calcul d'ANI, incluant, sans s'y limiter, OrthoANI (Lee et al, Int J Syst Evol Microbiol. 2016, 66(2):1100-1103) et JSpeciesWS (Richter et al, Bioinformatics, 2016, 32(6):929-931). Typiquement, les génomes de souches bactériennes appartenant à la même espèce présentent une ANI supérieure ou égale à 95% tandis que les génomes de souches bactériennes appartenant au même genre présentent une ANI supérieure ou égale à 80% (Jain et al, Nat Commun. 2018; 9: 5114).

La séquence génomique de la souche *Brevundimonas massiliensis* sp. nov. déposée à la CNCM le 13 janvier 2021 sous le numéro I-5641 a été obtenue par séquençage de novo permettant la génération de 8 scaffolds, présentés dans le listing de séquences comme les séquences SEQ ID NO : 2 à 9. La taille de séquence obtenue par alignement des 8 scaffolds est compatible avec la taille des génomes du genre *Brevundimonas* et est donc représentative du génome entier.

Les séquences SEQ ID NO : 2 à 9 permettent ainsi la comparaison par paire de séquences bactériennes sur génome entier, pour le calcul de l'ANI.

De manière préférée, la bactérie présente une identité nucléotidique moyenne (ANI) d'au moins 95%, de préférence au moins 95,5%, de préférence au moins 96%, ou au moins 96,5%, au moins 97%, au moins 97,5%, au moins 98%, au moins 98,5%, au moins 99%, au moins 99,5%, voire au moins 99,8% par rapport au génome complet de la souche déposée auprès de la CNCM le 13 janvier 2021 sous le numéro I-5641.

L'appartenance d'une bactérie à cette espèce peut être en outre confirmée par une hybridation ADN-ADN (DDH), notamment *in silico,* d'au moins 70% entre l'ADN génomique de la bactérie et l'ADN génomique de la souche B. *massiliensis* sp. nov. déposée à la CNCM sous le numéro I-5641 (Goris et al, Int. J. of Systematic and Evolutionary Microbiology, 2007, 57, 81-91).

Au sens de la présente invention on entend par « peau saine », une zone de peau sur laquelle est appliquée la bactérie selon l'invention et dite « non pathologique » par un dermatologue, c'est-à-dire ne présentant pas d'infection, de cicatrice, de maladie ou d'affection cutanée telle que candidose, impétigo, psoriasis, eczéma, acné ou dermatite, ou de plaies ou de blessures.

Au sens de la présente invention, on entend d'un point de vue cosmétique un effet de prévention du vieillissement cutané prématuré ou accéléré, par la capacité de lutter, prévenir les signes de vieillissement cutané induit par l'environnement, notamment le stress, l'alimentation ou la pollution, d'un vieillissement cutané photo-induit, de prévention de l'apparition de tâches pigmentaires, d'amélioration de l'homogénéité du teint et/ou d'amélioration de la production de sébum. Plus généralement, il s'agit de prévenir les effets d'une exposition à un ou plusieurs facteurs internes et/ou externes de vieillissement cutané. Les expositions peuvent être typiquement des facteurs environnementaux en lien avec le mode de vie des sujets, à savoir l'exposition aux rayonnements solaires, la pollution de l'air, le tabagisme et l'alimentation. On peut aussi parler d'exposome, qui regroupe la totalité des expositions auxquelles un individu est sujet de sa conception à sa mort, c'est-à-dire l'ensemble des facteurs externes et internes, leurs interactions, et la réponse du corps humain se traduisant par des signes biologiques et cliniques.

Dans un mode de réalisation particulier, le vieillissement peut être typiquement un vieillissement photo-induit, c'est-à-dire dû aux rayonnements solaires comprenant les rayonnements UV, les rayonnements infrarouges, ainsi que la lumière visible.

### Méthodes de suivi et de criblage

Les résultats obtenus mettent en évidence le potentiel de la nouvelle espèce de l'invention en tant que marqueur d'un vieillissement cutané prématuré. La présence de ladite bactérie à la surface d'une peau saine est corrélée à une peau sans taches et/ou à un teint plus homogène et/ou à un taux de sébum plus élevé.

A ce titre, un objet de l'invention concerne une méthode de criblage de substances susceptibles de présenter un effet cosmétique, notamment un effet de prévention du vieillissement cutané prématuré, en particulier photo-induit.

Il peut s'agir par exemple de petites molécules synthétiques, seules ou en combinaison, ou d'extraits naturels, notamment d'extraits naturels de plantes.

Cette méthode comprend l'application topique des substances candidates sur une zone de la peau, et la détermination ou suivi de la présence et/ou de la quantité de bactéries de cette nouvelle espèce.

La détermination de la présence et/ou quantité de bactéries à la surface de la peau peut être réalisée de manière avantageuse par amplification de l'ADN génomique bactérien, par exemple par PCR quantitative (qPCR).

Selon un mode de réalisation particulier, le gène de la protéine transporteur de soufre ThiS est amplifié, par exemple en utilisant les amorces de PCR décrites dans l'exemple 2.

Une substance candidate qui stimule la prolifération de la bactérie à la surface de la peau est identifiée comme susceptible de présenter un effet cosmétique. De manière avantageuse, l'effet cosmétique est un effet de prévention d'un vieillissement cutané prématuré ou accéléré, d'un vieillissement cutané induit par l'environnement, notamment le stress, l'alimentation ou la pollution, d'un vieillissement cutané photo-induit, de prévention de l'apparition de tâches pigmentaires, d'amélioration de l'homogénéité du teint et/ou d'amélioration de la production de sébum

L'état de vieillissement prématuré d'une peau humaine peut être également évalué, en déterminant la présence et/ou la quantité de bactéries de cette nouvelle espèce de l'invention, sur une zone de peau saine, la présence de ladite bactérie étant corrélée à une peau jeune sans taches et/ou à un teint plus homogène et/ou à un taux de sébum plus élevé. En d'autres termes, la quantité de bactéries trouvée à la surface de la peau diminue tandis que la peau présente un état de vieillissement prématuré de plus en plus marqué.

Les exemples et figures illustrent l'invention, sans limiter la portée de l'invention.

### EXEMPLES

### Exemple 1 : Isolement d'une nouvelle espèce bactérienne (étude clinique)

### Matériel et méthodes

### 1- Inclusion des volontaires

Les prélèvements de peau ont été collectés auprès de 47 femmes en bonne santé vivant autour de Marseille dans deux tranches d'âge différentes : de 18 à 30 ans (groupe jeune) et de plus de 55 ans (groupe âgé).

De plus pour cette étude, des conditions supplémentaires de recrutement ont été ajoutées : ne pas avoir suivi de traitement antibiotique ou antihistaminique pendant au moins 1 mois, ne pas souffrir d'une pathologie cutanée telle que dermatite atopique ou psoriasis (déterminée par un examen dermatologique), ne pas avoir pris une douche ou s'être lavé le front depuis la veille (au moins 12 heures) et ne pas avoir appliqué de produit cosmétique depuis le dernier lavage sur la zone prélevée. Pour le groupe jeune, les femmes devaient suivre un traitement contraceptif, à l'exclusion des pilules hautement dosées ayant une action connue sur l'acné. Les participantes du groupe âgé devaient être ménopausées, ne devaient suivre aucun traitement hormonal substitutif et devaient présenter des signes de photo vieillissement (taches sur le visage, le teint jaune, les rides marquées etc.).

Les résultats obtenus de 47 volontaires pour l'analyse des échantillons du front, sont répartis en 23 volontaires du groupe jeune et 24 volontaires du groupe âgé.

### 2- Prélèvement des échantillons de microbiote cutané

Les zones de front échantillonnées étaient de 10 cm². Les prélèvements ont été effectués en utilisant des écouvillons stériles imbibés du milieu de transport « Culture Top^{®} Transport Media » (C-top Ae-Ana, Eurobio, France) et de manière Z-Stroked. 2 écouvillons ont été utilisés simultanément pour la culturomique. Des écouvillons agités à l'air ont été réalisés comme contrôle négatif. Les échantillons ont été immédiatement mis en culture pour la culturomique. Après prélèvement, ces échantillons sont immédiatement conservés à -80 °C avant l'extraction d'ADN.

### 3- Identification des micro-organismes par spectrométrie de masse MALDI-TOF

Après la mise en culture, les micro-organismes isolés ont été identifiés à l'aide de la technique MALDI-TOF. Les bactéries ont été directement déposées sur la cible MSP96 (Bruker Daltonics, GmbH, Brême, Allemagne) avec un contrôle positif (*Escherichia coli*) un contrôle négatif (la matrice), cette méthode est appelée « dépôt direct ». L'acquisition des données a été réalisée sur le MALDI Biotyper (système Microflex LT ; Bruker Daltonics GmbH, Brème, Allemagne) à l'aide du logiciel Flex ControlTM et du logiciel d'identification MALDI BioTyper RTC (Bruker Daltonics GmbH, Brème, Allemagne). Lorsque l'identification n'est pas rendue possible par cette technique, trois repiquages successifs de la bactérie sont réalisés de manière à la purifier au maximum. Pour chaque nouvel essai d'identification, une extraction à l'acide formique est réalisée, 1µL de surnageant est déposé sur la plaque. Cette méthode permet de générer un spectre protéique sans impureté et maximise les chances de rapprocher le spectre généré avec les spectres contenus dans la base de données.

### 4- Identification des micro-organismes par séquençage génomique

Dans le cas de nouveaux échecs d'identification par la technique de MALDI-TOF, le séquençage via les méthodes de Next Génération Sequencing est réalisé avec l'IlluminaMiseq (Illumina Inc., San Diego, CA, USA) et la méthode Oxford Nanopore (Oxford Nanopore Technologies, Inc, Oxford, UK).

Séquençage du génome de *Brevundimonas massiliensis* sp. nov.

L'ADN génomique (ADNg) de *Brevundimonas massiliensis* sp. nov. (souche de l'invention) a été extrait en deux étapes : un traitement mécanique a d'abord été réalisé avec des billes de verre lavées à l'acide (G4649 Sigma, St Louis, MO, USA) à l'aide de l'appareil FastPrep-24 ^{™} 5G Grinder (mpBio, Santa Ana, CA, USA) avec une vitesse de rotation maximale (6.5 m/s) pendant un temps de 90s. Ensuite, après 30 minutes d'incubation de lysozyme à 37 °C, l'ADN a été extrait sur le biorobot EZ1 (QIAGEN, Valencia, CA, USA) avec le kit EZ1 DNA Tissues. L'ADNg de *Brevundimonas massiliensis* sp. nov. a été quantifié par un test Qubit haute sensibilité (Life technologies, Carlsbad, CA, USA) à 0,2 ng/µl. L'ADN génomique a ensuite été séquencé avec la technologie MiSeq (Illumina Inc, San Diego, CA, USA). Pour préparer la bibliothèque d'extrémité appariée (« pair ends »), une dilution a été effectuée pour avoir 1 ng du génome comme matrice d'entrée pour préparer la bibliothèque d'extrémité appariée. L'étape de « tagmentation » a permis de fragmenter et de marquer l'ADN via l'ancrage au niveau des extrémités cohésives débordantes d'une séquence de nucléotides universelle. Ensuite, une amplification par PCR à cycle limité (12 cycles) introduit des codes-barres à double index. Après purification sur billes AMPure XP (Beckman Coulter Inc, Fullerton, CA, USA), les banques ont ensuite été normalisées sur billes spécifiques selon le protocole Nextera XT (Illumina Inc., San Diego, CA, USA). Les bibliothèques normalisées ont été regroupées en une seule bibliothèque pour le séquençage sur l'appareil MiSeq. La génération automatisée de cluster et le séquençage des extrémités appariées avec des lectures d'index doubles ont été effectués en une seule exécution de 39 heures à 2x250 pb. Des informations totales de 4,5 Gb ont été obtenues à partir d'une densité d'agrégats de 462 K / mm2 avec un cluster passant des filtres de contrôle de qualité de 93,9%. Les 9 045 583 lectures finales appariées ont été filtrées en fonction des qualités de lecture. De sorte à améliorer la séquence générée par la technique Illumina, l'approche Oxford Nanopore a été réalisée pour le séquençage de l'ADN génomique via la méthode 1D via la technologie Minlon en utilisant le kit SQK-LSK109. La bibliothèque a été construite à partir de 1 µg d'ADN génomique sans fragmentation, ni réparation d'extrémité. Les adaptateurs ont été ligaturés aux deux extrémités de l'ADN génomique. Après purification sur billes AMPure XP (Beckman Coulter Inc, Fullerton, CA, USA), la librairie a été quantifiée par un test Qubit haute sensibilité (Life technologies, Carlsbad, CA, USA). 1353 pores actifs ont été détectés pour le séquençage et le flux de travail WIMP a été choisi pour l'analyse bio-informatique en direct. 655K reads en tant que données brutes ont été générées.

### Analyse du génome

L'annotation a été obtenue avec le logiciel Prokka Galaxy Version 1.14.5+galaxy0 en utilisant les paramètres par défaut à l'aide de la plateforme Online Galaxy. Les données de séquence du génome ont été téléchargées sur le Type (Strain) Genome Server (TYGS), qui est une plate-forme de bio-informatique gratuite disponible en ligne (https://tygs.dsmz.de) pour effectuer une analyse taxonomique complète basée sur le génome. La détermination des génomes de souches type (« type strain » = souche officielle) les plus proches a été effectuée de deux manières complémentaires. Tout d'abord, le génome de *Brevundimonas massiliensis sp. nov.* (souche de l'invention) a été comparé à tous les génomes de souches types disponibles dans la base de données TYGS via l'algorithme MASH, qui permet une approximation rapide de la parenté inter génomique, et le choix des dix souches types avec les plus petites distances. Deuxièmement, un ensemble supplémentaire de dix souches types étroitement apparentées a été déterminé via les séquences génétiques de l'ADNr 16S. Celui-ci a été extrait du génome soumis en utilisant RNAmmer et chaque séquence a ensuite été BLASTée contre la séquence du gène de l'ADNr 16S de chacune des 12983 souches de type actuellement disponibles dans la base de données TYGS. La superposition des résultats de ces deux méthodes a permis d'obtenir le classement des 50 meilleures souches de type correspondant (selon le bit score) et pour calculer ultérieurement des distances précises en utilisant l'approche Genome BLAST Distance Phylogeny (GBDP) avec l'algorithme « couverture » et la formule de distance d5. Ces distances ont finalement été utilisées pour déterminer les 10 génomes de souches de type les plus proches pour chacun des génomes utilisateurs. Toutes les comparaisons par paires parmi l'ensemble de génomes ont été effectuées en utilisant le GBDP et des distances inter génomiques précises déduites avec l'algorithme « rognage » (Trimming) et la formule de distance d5. Les calculs ont été effectués avec 100 itérations. Les valeurs numériques DDH et les intervalles de confiance ont été calculés en utilisant les paramètres recommandés par le logiciel GGDC v2.1. En complément, le degré de similarité génomique de la souche de l'invention avec des espèces proches a été estimé à l'aide du logiciel OrthoANI en utilisant les paramètres par défaut. Les gènes de résistance aux antibiotiques et la présence de protéines liées à la pathogenèse ont été étudiés à l'aide des outils ABRicate intégrant les bases de données CARD et VFDB à l'aide de la plateforme Online Galaxy. L'analyse de la synthèse de métabolites secondaires a été effectuée par le logiciel AntiSmash puis confortée par l'analyse des séquences protéiques de la base de données Kyoto Encyclopedia of Genes and Genomes Pathway (KEGG) en utilisant BLASTp avec un seuil de valeur e de 1E⁻³.

### RESULTATS

### 1- Isolement et identification d'une nouvelle espèce du microbiote cutané

128 espèces différentes ont pu être isolées à partir des prélèvements issus du front, réparties dans 55 genres différents. Les genres majoritaires du front identifiés sont *Staphylococcus* (isolés sur 100% des volontaires), *Cutibacterium* (76,60%) et *Streptococcus* (48,94%) dont les principaux représentants *Staphylococcus epidermidis, Cutibacterium acnes, Streptococcus mitis*/ *oralis.*

Parmi elles, une nouvelle espèce a pu être isolée sur la peau d'une volontaire de 60 ans. Le séquençage de son génome a révélé qu'il s'agissait d'une nouvelle espèce du genre *Brevundimonas* jamais décrite et nommée *Brevundimonas massiliensis* sp. nov.

La souche cultivable de *Brevundimonas massiliensis* sp. nov. a été déposée à la CNCM (collection nationale de culture de microorganismes de l'institut Pasteur) sous le numéro CNCM-I-5641.

### Exemple 2 : Etude de corrélations entre la présence de Brevundimonas massiliensis sp. nov. et les paramètres cliniques

### Objectif

L'objectif principal de l'étude était d'analyser chez la femme adulte la présence de bactéries de l'espèce *Brevundimonas massiliensis* sp. nov. sur la peau du visage. L'objectif secondaire était d'étudier les variations de cette espèce bactérienne en fonction de l'âge et des caractéristiques cliniques de la peau.

### Matériel et méthodes

### Etude clinique

Des prélèvements du microbiote cutané ont été réalisés sur le front de 108 sujets volontaires répartis en 52 volontaires du groupe jeune (18-30 ans) et 56 volontaires du groupe âgé (55-70 ans). A partir de ces prélèvements, une détection des bactéries de l'espèce *Brevundimonas massiliensis* sp. nov. a été réalisée, en utilisant la technique de PCR quantitative.

Des évaluations cliniques de l'état de la peau et des mesures de paramètres cliniques tels que le taux de sébum, le taux d'hydratation, la perte insensible en eau et les mesures des propriétés biomécaniques de la peau ont été menées sur le visage de l'ensemble des sujets volontaires.

Des analyses de corrélations ont été réalisées entre les données cliniques, l'âge des sujets volontaires et la quantité de bactéries de l'espèce *Brevundimonas massiliensis* sp. nov. trouvées à la surface de la peau du visage.

### Paramètres d'évaluation

### Prélèvement du microbiote cutané

Le microbiote cutané a été prélevé sur une surface de 25 cm² au niveau du front. Les prélèvements ont été effectués à l'aide d'un écouvillon (Transwab^{®}) pour chaque zone, en frottant délicatement l'écouvillon sur la peau du front.

### Prise de photos

Des photographies numériques à haute résolution du visage ont été réalisées à l'aide d'une table de repositionnement HeadScan et du logiciel CameraScan (Orion Concept), au laboratoire COSNAT Provence. Les acquisitions standardisées des sujets volontaires ont été réalisées par un/une technicien(ne) clinique, de face et de profil (45° à gauche et 45° à droite) et avec les filtres polarisés croisés et polarisés parallèles, lors de la visite d'évaluation. Les acquisitions ont ensuite servi de support visuel pour les scorages cliniques réalisés à la fin de l'étude.

### Mesures cliniques

Certaines mesures cliniques sont des mesures instrumentales non invasives nécessitant un contact cutané. Elles ont été effectuées sur une zone du front distincte de la zone prélevée pour le microbiote, ou sur la tempe pour les propriétés mécaniques :
- Hydratation par Cornéométrie (Corneometer^{®} CM825 - COURAGE & KHAZAKA)
- Taux de sébum par Sébumétrie (Sebumeter^{®} SM 815 - COURAGE & KHAZAKA)
- Perte insensible en eau par Tewamétrie (Tewametre^{®} TM300)
- Propriétés biomécaniques de la peau par Cutométrie (Cutometer^{®} MPA 580 - COURAGE & KHAZAKA)

### Scorages cliniques

Les scorages cliniques ont été réalisés par les investigateurs dermatologues sur la base des photographies standardisées, réalisées pendant la visite d'inclusion. Les échelles de scorage utilisées sont les suivantes :
- Rides de la patte d'oie selon l'Atlas BAZIN, Atlas du vieillissement cutanée, Volume 1 p. 40, éditions MED'COM, 2007,
- Ridules sous oculaires selon l'Atlas BAZIN, Atlas du vieillissement cutanée, Volume 1 p. 40, éditions MED'COM, 2007,
- Taches pigmentaires selon l'Atlas BAZIN, Skin Aging Atlas, Volume 2 p.93, MED'COM publishing 2010 pour le visage,
- Homogénéité du teint (échelle interne) selon l'échelle suivante en 5 points : 1- teint très hétérogène, 2- teint peu homogène, 3- teint moyennement homogène, 4- teint homogène, 5- teint très homogène
- Eclat du teint (échelle interne) selon l'échelle suivante en 5 points : 1- teint terne, éteint, 2-teint peu éclatant et lumineux, 3- teint moyennement éclatant et lumineux, 4- teint éclatant et lumineux, 5- teint très éclatant et lumineux

### Analyse de Brevundimonas massiliensis sp. nov. par PCR quantitative

Tous les échantillons de microbiote cutané ont été congelés et stockés à -80 °C immédiatement après prélèvement. Après décongélation, 20 µl de Protéinase K à 20 mg/ml ont été ajoutés à chaque échantillon et chauffés 1h à 50 °C au bain-marie puis concentrés au SpeedVac avant extraction de l'ADN à l'aide du kit QIAamp^{®} PowerFecal^{®} Pro DNA (Qiagen). Une PCR quantitative a ensuite été réalisée, grâce aux systèmes PCR spécifiques du génome de *Brevundimonas massiliensis* sp. nov. (Tableau 1) et du TaqMan^{®} Fast Advanced Master Mix (Applied Biosystems). La réaction de PCR est réalisée grâce à l'appareil RT-PCR QuantStudio^{™} 7 Pro (Applied Biosystems) sur 50 cycles d'amplification.

Le système qPCR a été designé en utilisant le logiciel PrimerBlast+ et vérifié en utilisant l'outil BlastN contre la base de données nr. Le tableau 1 ci-dessous décrit le système PCR, comprenant un couple d'amorces spécifiques et une sonde, conçu pour amplifier spécifiquement l'ADN génomique de *Brevundimonas massiliensis* sp. nov. en ciblant le gène de la protéine transporteur de soufre ThiS impliquée dans la synthèse de thiamine.

**Tableau 1 : Système qPCR conçu pour amplifier spécifiquement l'ADN de Brevundimonas massiliensis sp. nov.**

| **Bactérie cible** | **Gène cible** | **Amorce sens** | **Amorce antisens** | **Sonde** |
|---|---|---|---|---|
| ***Brevundimonas massiliensis* sp. nov. souche de l'invention** | ThiS (sulfur carrier protein ThiS) | GCATTCAAGTC AACGGCGAT (SEQ ID NO: 10) | CGAACTGAACCAGC TCGATG (SEQ ID NO: 11) | TGAAGGCGCTG TCGCTCGAC (SEQ ID NO: 12) |

### Analyse statistique

Les données cliniques ont été formatées et l'absence d'erreur vérifiée à l'aide du logiciel R v 4.2.1. Toutes les analyses statistiques ont été réalisées dans le logiciel R. L'analyse de variations cliniques entre les groupes de sujets jeunes et âgés a été réalisée pour toutes les variables continues en utilisant le test Wilcoxon signed-rank test, et les p-values ont été ajustées par la méthode Benjamini-Hochberg. Les corrélations entre variables continues ont également été recherchées en utilisant le coefficient de corrélation Pearson et les résultats ajustés selon la même méthode. Les potentielles valeurs aberrantes ont été recherchées en utilisant le test du Z-score.

Un modèle de régression multilinéaire a ensuite été construit, basé sur les variables continues (données cliniques et valeurs de PCR quantitative) en utilisant la fonction 'lm' du logiciel R et les paramètres 'age ~ all continuous variables' afin d'observer quelles variables expliquent le plus les différences observées entre les groupes d'âge différent et prendre en compte tout élément confondant.

Nous avons ensuite comparé les résultats de qPCR avec les variables cliniques (test apparié) avec le test de Wilcoxon, en ajustant les p-values comme précédemment. Pour les variables continues, nous avons recherché les corrélations entre la qPCR et la variable grâce à la corrélation de Spearman sur la population globale.

### Résultats

### Caractérisation de la population étudiée

L'analyse des caractéristiques cliniques de la population étudiée révèle que les deux groupes de sujets (groupe jeune et groupe âgé) sont significativement différents pour toutes les variables étudiées, à l'exception des valeurs de téwamétrie et de cornéométrie (Tableau 2). Ainsi, les sujets des deux groupes ne présentent pas de différences en termes de barrière cutanée (téwamétrie) ou d'hydratation cutanée (cornéométrie), deux caractéristiques cliniques témoignant d'une peau saine. En revanche, les sujets âgés présentent de manière significative : une moindre production de sébum, plus de taches pigmentaires, un teint plus terne et plus hétérogène, plus de rides sous-oculaires et au niveau de la patte d'oie (Tableau 2). Les mesures au cutomètre révèlent également que la peau des sujets du groupe âgé est en moyenne significativement moins ferme (valeurs R6 en hausse, témoignant d'une plus grande viscosité de la peau, associée à une perte en collagène) et moins élastique (valeur R1 en hausse (moins bon retour élastique) et valeurs R2, R5 et R7 (paramètres d'élasticité) en baisse) (Tableau 2).

Ces données témoignent du vieillissement cutané.

**Tableau 2 : Analyse statistique des variations cliniques entre les groupes de sujets jeunes et âgés. Les variations sont exprimées en pourcentage pour les mesures instrumentales ou en point pour les scorages cliniques (les scores pouvant aller de 1 à 5) (test Wilcoxon signed-rank test avec ajustement des valeurs de p par la méthode Benjamini-Hochberg). Le seuil de significativité est fixé à 0,05 de la valeur de p. ****p<0,0001.**

| **Variable** | **Variation dans le groupe âgé par rapport au groupe jeune** | **Valeur de p ajustée (BH)** | **Analyse statistique** |
|---|---|---|---|
| Tewamétrie | -8,20% | 0.11475 | non significatif |
| Cornéométrie | 5,20% | 0.135 | non significatif |
| Sébumétrie | -17,20% | 3.65E-06 | **** |
| Taches pigmentaires | 3 | 6.90E-16 | **** |
| Eclat du teint | -1 | 6.08E-16 | **** |
| Homogénéité du teint | -1 | 5.87E-10 | **** |
| Sévérité des rides de la patte d'oie | 3,2 | 3.18E-18 | **** |
| Sévérité des rides sous oculaires | 2,6 | 1.56E-17 | **** |
| cutométrie R0 | -12,6% | 0.000372 | **** |
| cutométrie R1 | -41,7% | 3.17E-12 | **** |
| cutométrie R2 | -38,1% | 2.57E-15 | **** |
| cutométrie R3 | -12,6% | 0.000372 | **** |
| cutométrie R4 | -41,7% | 3.17E-12 | **** |
| cutométrie R5 | -44,0% | 1.45E-15 | **** |
| cutométrie R6 | -47,3% | 5.29E-13 | **** |
| cutométrie R7 | -51,6% | 2.08E-16 | **** |
| cutométrie R8 | -47,3% | 5.61E-13 | **** |

Présence, évolution et corrélations statistiques entre la quantité de *Brevundimonas massiliensis* sp. nov. et les paramètres cliniques

Les échantillons n'atteignant pas le seuil de détection (Ct) avant les 50 cycles de qPCR ont été considérés comme négatifs (bactérie indétectable).

La bactérie est considérée comme commensale puisqu'elle a été détectée sur la peau de 96% des volontaires du groupe jeune (53 sur 55) et 89% des volontaires du groupe âgé (49 sur 55).

La quantité de *Brevundimonas massiliensis* sp. nov. détectée par qPCR a été corrélée aux paramètres cliniques. Ces résultats sont résumés dans le Tableau 3. Ils démontrent que *Brevundimonas massiliensis* sp. nov. diminue de manière significative avec l'augmentation du score de taches pigmentaires. Une plus grande quantité de *Brevundimonas massiliensis* sp. nov. est associée à une plus grande homogénéité du teint est une production de sébum plus élevée. La quantité de *Brevundimonas massiliensis* sp. nov. n'est pas corrélée de manière significative avec les variations des paramètres d'hydratation (cornéométrie), de barrière cutanée (téwamétrie) ou des propriétés biomécaniques de la peau.

Bien que *Brevundimonas massiliensis* sp. nov. présente une tendance à la diminution en fonction de l'âge des volontaires, cette corrélation n'est pas significative (p=0,061). Cette observation pourrait s'expliquer par une hétérogénéité du groupe âgé, certaines volontaires présentant moins de tâches pigmentaires que d'autres, paramètre auquel est corrélée la quantité de la bactérie.

De manière intéressante, lorsque l'analyse statistique est réalisée au sein du groupe âgé comprenant 56 volontaires entre 55 et 70 ans et présentant un nombre plus ou moins important de tâches pigmentaires, l'association entre la quantité de la bactérie *Brevundimonas massiliensis* sp.nov. et un score de tâches pigmentaires plus faible est renforcée avec un coefficient de corrélation de -0,41 et une p-value de 0,0018. Ce résultat indique que la présence de tâches pigmentaires est le paramètre qui est le plus associé à la diminution de la bactérie à la surface de la peau, indépendamment de l'âge des individus.

Ces résultats confirment que *Brevundimonas massiliensis* sp. nov. est associée aux peaux présentant une production de sébum plus élevée, un teint plus homogène et présentant moins de tâches pigmentaires.

**Tableau 3 : Evolution de la quantité de Brevundimonas massiliensis sp. nov. en fonction des paramètres cliniques. Les coefficients de corrélation de Spearman sont indiqués ainsi que leur significativité pour chaque paramètre. ns=non significatif, *p<0,05, **p<0,01,***p<0,001, ****p<0,0001.**

| | **Variation de la quantité de *Brevundimonas massiliensis* sp. nov. en fonction du paramètre** | **Coefficient de corrélation R** | **Significativité (valeur de p) Spearman** |
|---|---|---|---|
| Taches **pigmentaires** | Diminution | -0,22 | * p=0,024 |
| **Homogénéité du teint** | Augmentation | 0,21 | * p=0,032 |
| **Sébumètre** | Augmentation | 0,24 | *p= 0,012 |
| **Âge** | Aucune | -0,18 | p= 0,061 |
| **Eclat du teint** | Aucune | 0,13 | ns p=0,18 |
| **Rides de la patte d'oie** | Aucune | -0,13 | ns p=0,19 |
| **Cornéomètre** | Aucune | -0,13 | ns p=0,19 |
| **Tewamètre** | Aucune | -0,03 | ns p=0,76 |
| **Rides sous-oculaires** | Aucune | -0,15 | ns p=0,11 |
| **R0** | Aucune | 0,07 | ns p=0,47 |
| **R1** | Aucune | -0,024 | ns p=0,81 |
| **R2** | Aucune | 0,081 | ns p=0,4 |
| **R3** | Aucune | 0,07 | ns p=0,47 |
| **R4** | Aucune | -0,024 | ns p=0,81 |
| **R5** | Aucune | 0,1 | ns p=0,28 |
| **R6** | Aucune | -0,054 | ns p=0,58 |
| **R7** | Aucune | 0,1 | ns p=0,29 |
| **R8** | Aucune | 0,11 | ns p=0,25 |

## Revendications

1. Bactérie appartenant à l'espèce *Brevundimonas massiliensis sp. nov.* **caractérisée en ce que** son génome i) présente une identité nucléotidique moyenne (ANI) d'au moins 95% par rapport au génome complet de la souche déposée auprès de la CNCM le 13 janvier 2021 sous le numéro I-5641 et ii) présente au moins 98,65% d'identité avec la séquence nucléotidique SEQ ID NO : 1 (gène 16S *B.massiliensis sp. nov.).*

2. Bactérie selon la revendication 1, **caractérisée en ce qu'**il s'agit de la souche déposée auprès de la CNCM le 13 janvier 2021 sous le numéro I-5641.

3. Méthode de criblage de substances susceptibles de présenter un effet cosmétique, ladite méthode comprenant la mise en contact d'une peau humaine saine avec une substance candidate, et le suivi de la présence et/ou de la quantification d'une bactérie de l'espèce *Brevundimonas massiliensis sp. nov.* à la surface de la peau, par ce en quoi une substance qui stimule la prolifération de la bactérie à la surface de la peau est identifiée comme susceptible de présenter un effet cosmétique, l'espèce *Brevundimonas massiliensis sp. nov.* étant **caractérisée en ce que** son génome i) présente une identité nucléotidique moyenne (ANI) d'au moins 95% par rapport au génome complet de la souche déposée auprès de la CNCM le 13 janvier 2021 sous le numéro I-5641 et ii) présente au moins 98,65% d'identité avec la séquence nucléotidique SEQ ID NO : 1 (gène 16S *B.massiliensis sp. nov.*) ; ladite bactérie étant de préférence la souche déposée à la CNCM le 13 janvier 2021 sous le numéro I-5641.

4. Méthode de criblage selon la revendication 3, dans laquelle l'effet cosmétique est un effet de prévention d'un vieillissement cutané prématuré ou accéléré, d'un vieillissement cutané induit par l'environnement, notamment le stress, l'alimentation ou la pollution, d'un vieillissement cutané photo-induit, de prévention de l'apparition de tâches pigmentaires, d'amélioration de l'homogénéité du teint et/ou d'amélioration de la production de sébum.

5. Méthode selon l'un quelconque des revendications 3 ou 4, comprenant une quantification de ladite bactérie par PCR quantitative.

6. Utilisation d'une bactérie de l'espèce *Brevundimonas massiliensis sp. nov.,* comme marqueur d'un vieillissement cutané prématuré ou accéléré, étant entendu que la présence de ladite bactérie est corrélée à une peau protégée contre un vieillissement cutané prématuré ou accéléré; l'espèce *Brevundimonas massiliensis sp. nov.* étant **caractérisée en ce que** son génome i) présente une identité nucléotidique moyenne (ANI) d'au moins 95% par rapport au génome complet de la souche déposée auprès de la CNCM le 13 janvier 2021 sous le numéro I-5641 et ii) présente au moins 98,65% d'identité avec la séquence nucléotidique SEQ ID NO : 1 (gène 16S *B.massiliensis sp. nov.*); ladite bactérie étant de préférence la souche déposée à la CNCM le 13 janvier 2021 sous le numéro I-5641.
